# EUROPÄISCHE PATENTSCHRIFT

(11) **EP 2 536 816 B1**
(45) Veröffentlichungstag und Bekanntmachung des Hinweises auf die Patenterteilung: **10.01.2018**
(21) Anmeldenummer: 11716462.4
(22) Anmeldetag: 14.02.2011
(51) Int. Cl.: C12M 1/12, C12M 3/00, C12M 1/00

(54) **FOLIENSYSTEM ZUM BEREITSTELLEN EINES BIOREAKTORS UND ZUGEHÖRIGE FOLIENSYSTEMROLLE SOWIE VERFAHREN ZUM HERSTELLEN EINES BIOREAKTORS, BIOREAKTOR UND BIOREAKTORSYSTEM**
FILM SYSTEM FOR THE PROVISION OF A BIOREACTOR AND ASSOCIATED FILM SYSTEM ROLL AND METHOD FOR PRODUCING A BIOREACTOR, BIOREACTOR AND BIOREACTOR SYSTEM
SYSTÈME DE FEUILLES POUR CONSTITUER UN BIORÉACTEUR, ROULEAU DE SYSTÈME DE FEUILLES, PROCÉDÉ POUR FABRIQUER UN BIORÉACTEUR, BIORÉACTEUR ET SYSTÈME DE BIORÉACTEUR

(30) Priorität: 15.02.2010 DE 102010008093
(43) Veröffentlichungstag der Anmeldung: 26.12.2012
(73) Patentinhaber: Phytolutions GmbH, 28759 Bremen (DE)
(72) Erfinder: THOMSEN, Claudia, 28757 Bremen (DE)
(74) Vertreter: Weidner Stern Jeschke
(86) Internationale Anmeldenummer: PCT/DE2011/000135
(87) Internationale Veröffentlichungsnummer: WO 2011/098076

(56) Entgegenhaltungen:
- WO-A1-2005/121309
- WO-A1-2009/059111
- WO-A2-2008/033573
- DE-A1- 10 164 458
- DE-A1-102008 023 368
- GB-A- 2 339 763
- US-A1- 2008 274 494
- DATABASE WPI Week 200122 Thomson Scientific, London, GB; AN 2001-213304 XP002648708, & JP 2000 320041 A (MAEDA KENSETSU KOGYO KK) 21. November 2000 (2000-11-21)

## Beschreibung

Die Erfindung betrifft ein Foliensystem zum Bereitstellen eines Bioreaktors zum Kultivieren photosynthetisch aktiver Organismen, insbesondere zum Kultivieren in wässrigen Lösungen, aus einem transparenten Folienmaterial, insbesondere aus einem Polyethylen, wobei das Foliensystem zwei einander gegenüberliegende Innenseiten und die einander gegenüberliegenden Innenseiten eine Trennverbindung aufweisen, sodass zwei separierte Bereiche ausgebildet sind, und die Trennverbindung unvollständig ist, sodass die zwei separierten Bereiche eine erste ungehinderte Verbindung aufweisen, wobei die gegenüberliegenden Innenseiten n unvollständige Trennverbindungen aufweisen, wobei n eine natürliche Zahl >1 ist, sodass n+1 separierte Bereiche ausgebildet sind, welche insbesondere durch die erste Verbindung und/oder durch eine zweite ungehinderte Verbindung durchgehend verbunden sind, dadurch gekennzeichnet, dass das Foliensystem zum Herstellen von Bioreaktoren variabler Länge einsetzbar ist, 'wobei das Foliensytem eine endlose Länge aufweist, 'wobei das endlose Foliensystem von einer transportablen Foliensystemrolle abrollbar ist sowie ein Verfahren zum Herstellen dieser Bioreaktoren.

Algen werden bei autotropher und mixotropher Kultivierung in sogenannten Phytobioreaktoren (PBRs) herangezogen und in großen Mengen produziert.

Es wird grundsätzlich zwischen starren Röhrensystemen aus Glas, PC, Plexiglas und flexiblen Foliensystemen unterschieden. Flexible Foliensysteme aus Polyethylen werden beispielsweise vertikal als Beutel aufgehängt. Verfügbare flexible Foliensysteme werden dem Verwender in Standardgrößen geliefert. Siehe hierzu zum Beispiel DE 10 200 023 368.

Für den Anwender wichtige Produktionsparameter bei dem Verwenden von PBRs sind eine möglichst große photosynthetische Oberfläche, welche im Idealfall mit einem geringen Lichtweg gekoppelt ist, was eine relativ und auch absolut hohe Produktivität gewährleisten kann. Weitere Anforderungen liegen in der Skalierbarkeit zum großindustriellen Maßstab, im Preis-Leistungs-Verhältnis und der universellen Einsetzbarkeit.

Die Aufgabe der Erfindung ist es, den Stand der Technik zu verbessern.

Gelöst wird die Aufgabe durch ein Foliensystem, insbesondere zum Bereitstellen eines Bioreaktors zum Kultivieren photosynthetisch aktiver Organismen, insbesondere zum Kultivieren in wässrigen Lösungen, aus einem transparenten Folienmaterial, insbesondere aus einem Polyethylen, wobei das Foliensystem zwei einander gegenüberliegende Innenseiten und die einander gegenüberliegenden Innenseiten eine Trennverbindung aufweisen, sodass zwei separierte Bereiche ausgebildet sind, wobei die Trennverbindung unvollständig ist, sodass die zwei separierten Bereiche eine erste ungehinderte Verbindung aufweisen.

Somit kann ein skalierbares Foliensystem bereitgestellt werden, aus dem Bioreaktoren fertigbar sind. Zudem erlaubt die Einfachheit des Aufbaus des Foliensystems eine kostengünstige Produktion des Bioreaktors.

Folgendes Begriffliche sei erläutert:
"Photosynthetisch aktive Organismen" sind beispielsweise Algen, welche insbesondere in Süßwasser oder Salzwasser kultiviert werden. Insbesondere können Algen sowohl eukariotische, pflanzenartige Organismen, welche Photosynthese betreiben, als auch Prokaryoten wie Blaualgen oder Grünalgen umfassen. Die Algen werden insbesondere so ausgewählt, dass diese beim Zurverfügungstellen von Licht und CO₂ möglichst viel Biomasse produzieren. Je nach Zielprodukt können Algen verwendet werden, welche insbesondere große Mengen des Zielprodukts bilden. Zielprodukte können beispielsweise Polysaccharide sein. Zudem kann beispielsweise die Algenbiomasse mit lipidhaltigen Algen in Biodiesel umgewandelt werden.

Eine "Folie" ist dadurch gekennzeichnet, dass diese eine große flächige Ausdehnung im Vergleich zu ihrer Dicke aufweist und somit eine Flexibilität hat, welche ein einfaches manuelles Umformen ermöglicht.

Ein "Folienmaterial" ist das Material, aus dem die Folie gefertigt wird. Es können insbesondere Kunststoffe wie Polyethylen zum Einsatz kommen.

Ein "transparentes" Folienmaterial zeichnet sich insbesondere dadurch aus, dass die Wellenlängen des Lichtes durchgelassen werden, welche zum Kultivieren des photosynthetisch aktiven Organismus' notwendig sind. Dabei kann die Folie sowohl als Kanten-, Breitband- oder inhomogener optischer Filter ausgestaltet sein.

Das "Foliensystem" kann in seiner einfachsten Form zusätzlich zu der Trennverbindung zwei Lagen Folien umfassen, welche aufeinander liegen. In diesem Fall bilden die sich berührenden Innenseiten der beiden Lagenfolien die "gegenüberliegenden Innenseiten".

Die "Trennverbindung" ist insbesondere so ausgestaltet, dass die Algen und die eventuell vorhandene Kultur- oder Nährflüssigkeit für die Algen die Trennverbindung nicht durchdringen können. Die Trennverbindung kann insbesondere durch Schweißen oder Kleben dem Foliensystem aufgeprägt werden.

Die "zwei separierten Bereiche" können insbesondere zwei Kammern umfassen, in welchen beispielsweise Algen in einer Suspension zum Zwecke der Zucht kultiviert werden.

Die "erste ungehinderte Verbindung" kann einen Übergang der Algen von dem ersten separierten Bereich mit dem zweiten separierten Bereich ermöglichen, wobei auch die Kultur- oder Nährflüssigkeit somit ausgetauscht werden kann. Durch die Trennverbindung kann die Oberfläche eines herzustellenden Bioreaktors derart erhöht werden, dass beispielsweise Algen vermehrt dem Sonnenlicht ausgesetzt sind.

Um ein Zirkulieren der photosynthetisch aktiven Organismen oder der Kultur- oder Nährflüssigkeit zu ermöglichen, kann die Trennverbindung derart ausgestaltet sein, dass die zwei separierten Bereiche eine zweite ungehinderte Verbindung aufweisen, welche durch die unvollständige Trennverbindung von der ersten unvollständigen Verbindung getrennt ist.

In einer Ausführungsform weisen die gegenüberliegenden Innenseiten n unvollständige Trennverbindungen auf, wobei n eine natürliche Zahl > 1 ist, sodass n + 1 separierte Bereiche ausgebildet sind, welche insbesondere durch die erste Verbindung und/oder zweite Verbindung durchgehend verbunden sind. Somit kann ein prinzipiell unendliches Foliensystem bereitgestellt werden, bei dem ein Benutzer die Anzahl der separierten Bereiche auf das für ihn vorliegende Problem anpassen kann. So kann der Benutzer entsprechend seiner örtlichen Gegebenheiten ein Teil des Foliensystems abtrennen und einen für ihn genau angepassten Bioreaktor herstellen.

In einer diesbezüglichen Ausbildung ist das Foliensystem zum Herstellen von Bioreaktoren variabler Länge einsetzbar, wobei das Foliensystem eine endlose Länge aufweist, wobei das endlose Foliensystem insbesondere von einer transportablen Foliensystemrolle abrollbar ist. Somit kann eine endlose Folie zum Ort des Aufstellens eines Bioreaktors transportiert werden und vor Ort in Bezug auf die Längen der Bioreaktoren variabel an die Bedingungen vor Ort angepasst werden.

"Endlos" in diesem Zusammenhang sind Foliensysteme, welche Längen weit über 50m aufweisen. Insbesondere umfasst der Begriff "endlos" Längen von 1.000m, 10.000m bis zu 100.000m.

Um schon vor dem Herstellen des eigentlichen Bioreaktors etwaige Versorgungsschläuche für den Bioreaktor bereitzustellen, kann das Foliensystem eine Versorgungseinrichtung oder mehrere Versorgungseinrichtungen aufweisen, insbesondere zum Versorgen der Algen mit CO₂, Rauchgas, Wärme, Kühlung oder Nährstoffen.

In einer diesbezüglichen Ausgestaltung ist die Versorgungseinrichtung als ein poröse Schlauch ausgestaltet. Somit kann schon beim Herstellen des Foliensystems eine Versorgungseinrichtung kostengünstig bereitgestellt werden. Der Versorgungsschlauch ist kann dabei aus dem identischen Material wie das Foliensystem hergestellt sein oder als separater Schlauch mit eingearbeitet werden. Zudem kann die Versorgungseinrichtung und somit der Schlauch etwas länger als das Foliensystem sein, sodass bei Abtrennen eines zukünftigen Bioreaktors Überstände für etwaige Anschlüsse bereitstehen.

Um eine möglichst günstige Bioreaktorenform bereit zu stellen, können die zwei Innenseiten je zwei Breitenaußenkanten und je zwei Längenaußenkanten aufweisen und die sich gegenüberliegenden Breitenaußenkanten der zwei gegenüberliegenden Innenseiten eine Außenkantenverbindung aufweisen, sodass insbesondere ein Folienschlauch mit einer Folienschlauchinnenfläche gebildet ist und die Folienschlauchinnenfläche im Wesentlichen die zwei Innenseiten umfasst. Somit werden die beiden Innenseiten zu einer Gesamtinnenseite überführt.

Folgendes begrifflich sei erläutert: die "Breitenaußenkanten" werden insbesondere durch die Foliensystemenden des Foliensystems an der kurzen Seite gebildet.

Die "Längenaußenkanten" können insbesondere durch das Ende des Foliensystems gebildet werden. Dabei verlaufen die Längenaußenkanten in einer besonderen Form parallel zu der Trennverbindung.

Eine "Außenverbindung" gewährleistet, dass insbesondere die Algen-Nährflüssigkeitsmischung durch diese Außenkantenverbindung begrenzt wird. Ein Durchdringen der Flüssigkeit oder der Algen durch die Außenkantenverbindung ist somit unterbunden.

Um möglichst handhabbare Bioreaktoren bereitstellen zu können, kann das Foliensystem eine Breite zwischen 0,2 m und 6 m aufweisen, wobei die Breite insbesondere einen Wert zwischen 0,5 m und 2 m beträgt.

In einer weiteren Ausführungsform weisen zwei benachbarte Trennverbindungen einen Abstand zwischen 2 cm und 200 cm auf, wobei der Abstand insbesondere einen Wert zwischen 5 cm und 20 cm aufweist. Somit können Kammern unterschiedlicher Größe bereitgestellt werden. Weiterhin können die Trennverbindungen äquidistant oder inhomogen verteilt ausgestaltet sein.

Um eine unterschiedliche Elastizität des Folienmaterials bereitstellen zu können, kann das Folienmaterial eine Foliendicke zwischen 0,1 mm und 100 mm aufweisen, wobei die Foliendicke insbesondere einen Wert zwischen 0,3 mm und 5 mm aufweist.

In einer Ausführungsform kann das Foliensystem eine Länge zwischen 0,5 m und 30.000 m aufweisen, wobei die Länge insbesondere einen Wert zwischen 2 m und 10.000 m aufweist. Somit kann eine "Endlos"-Rolle bereit gestellt werden. Insbesondere werden für das Foliensystem große Längen bevorzugt, sodass möglichst viele oder lange Photobioreaktoren aus einem Foliensystem hergestellt werden können.

Um Licht definiert in den Photobioreaktor einzuführen, kann das Folienmaterial eine Lichtleiter wie beispielsweise eine Glasfaser aufweisen, durch welchen insbesondere Licht in den Bioreaktor einkoppelbar ist. So kann zum einen der Lichtleiter an die Innenseiten geklebt sein und zum anderen selbst Bestandteil des Folienmaterials sein. Zudem kann das Folienmaterial Wölbungen zum Vergrößern der Oberfläche aufweisen.

In einer weiteren Ausprägungsform weist das Folienmaterial Nanopartikel und/oder Mikropartikel auf, welche auf physikalische und/oder chemische Eigenschaften des Folienmaterials einwirken.

Nanopartikel und/oder Mikropartikel können sowohl über ihre chemische Zusammensetzung als auch über ihre physikalische Form die physikalischen und/oder chemischen Eigenschaften des Folienmaterials ändern. Insbesondere können die Nanopartikel und/oder Mikropartikel zum Abschatten einzelner Wellenlängen oder Wellenlängenbereichen des Lichts eingesetzt werden. Weiterhin kann beispielsweise durch die Verwendung von Silber- oder Goldnanopartikeln eine Verstärkung des Lichts erfolgen. So kann diese Verstärkung beispielsweise auf die Plasmonenverstärkung an metallischen Oberflächen beruhen.

Um insbesondere eine Abschattung bereitstellen zu können, kann das Folienmaterial eine Beschichtung aufweisen. Somit kann auf das Folienmaterial beispielsweise eine weitere Folie mit speziellen optischen Eigenschaften aufgebracht werden.

Um durch die photosynthetisch aktiven Organismen nicht verwendetes Licht erneut den photosynthetisch aktiven Organismen zuzuführen, oder um bestimmte Wellenmengenbereiche des Lichtes ein- oder auszuschließen, kann das Folienmaterial und/oder die Beschichtung als ein Spektralfilter und/oder als ein teildurchlässiger Spiegel ausgestaltet sein.

In einem weiteren Aspekt der Erfindung wird die Aufgabe gelöst durch eine Foliensystemrolle, welche ein zuvor beschriebenes Foliensystem aufweist, welches auf der Foliensystemrolle aufgerollt ist, sodass einem Benutzer beliebig lange Foliensysteme als Einzelnutzen zum Herstellen eines Bioreaktors zur Verfügung stehen. Somit kann das zuvor beschriebene Foliensystem platzsparend gelagert und transportiert werden. Weiterhin kann das Foliensystem entsprechend der örtlichen Gegebenheiten für einen aufzubauenden Bioreaktor angepasst werden.

In einem weiteren Aspekt wird die Aufgabe gelöst durch ein Verfahren zum Herstellen eines Bioreaktors, wobei bei einem zuvor beschriebenen Foliensystem die Längenaußenkanten mediendicht verbunden werden, sodass eine Flüssigkeits-Algen-Mischung in dem Bioreaktor lagerbar ist. Durch diesen Schritt wird aus dem zuvor beschriebenen Foliensystem ein Bioreaktor.

In einer diesbezüglichen Ausführungsform wird vor dem mediendichten Verbinden ein Foliensystem von der Foliensystemrolle abgetrennt. Somit kann vor Ort ein speziell auf die Bedürfnisse angepasster Bioreaktor hergestellt werden.

Um die zu kultivierende Flüssigkeits-Algen-Mischung mit Nährstoffen und der entsprechenden Wärme zu versorgen, können vor dem mediendichten Verbinden ein oder mehrere Versorgungsschläuche in den Bioreaktor eingebracht werden.

In einem weiteren Aspekt der Erfindung wird die Aufgabe gelöst durch einen Bioreaktor, welcher nach dem vorstehend beschriebenen Verfahren hergestellt ist.

Zudem wird in einem weiteren Aspekt der Erfindung die Aufgabe gelöst durch einen Bioreaktor, welcher ein vorstehend beschriebenes Foliensystem aufweist, wobei eine oder beide Längenaußenkanten mediendicht verbunden sind, sodass insbesondere eine Flüssigkeitsalgenmischung in dem Bioreaktor kultivierbar ist.

Um die Algen bei optimaler Temperatur zu lagern, kann der Bioreaktor eine Temperiervorrichtung zum Kühlen oder Heizen der Flüssigkeits-Algen-Mischung aufweisen, wobei die Temperiervorrichtung insbesondere als ein Wärmeschlauch oder Kühlschlauch ausgestaltet ist, welcher mit der Flüssigkeits-Algen-Mischung im wärmetauschenden Kontakt steht.

In einer weiteren Ausführungsform weist der Bioreaktor ein Befestigungselement auf, insbesondere ein Befestigungsrohr, an welchem der Bioreaktor insbesondere hängend befestigt ist.

Um die Algen mit Nutzgasen zu versorgen, kann der Bioreaktor eine Begasungsvorrichtung aufweisen, welche insbesondere zum Begasen der Flüssigkeits-Algen-Mischung mit CO₂ oder Rauchgas dient, wobei die Begasungsvorrichtung insbesondere als ein poröser Begasungsschlauch ausgestaltet ist, welcher insbesondere von der Flüssigkeits-Algen-Mischung umgeben ist.

In einer weiteren Ausführungsform weist der Bioreaktor eine Erntevorrichtung auf, insbesondere zum Ernten der Algen aus der Flüssigkeits-Algen-Mischung, wobei die Erntevorrichtung insbesondere als Ernteschlauch ausgestaltet ist.

Ernten bezieht sich vorliegend insbesondere auf die Algen, welche beispielsweise in weiteren Verfahrensschritten umgewandelt werden. Das Ernten kann sich jedoch auch auf die Nährflüssigkeit oder auch auf von den Algen produzierten Stoffen beziehen. Die Nährflüssigkeit kann beispielsweise für eine weitere Kultivierung genutzt werden. Zu den von den Algen produzierten Stoffen zählen beispielsweise Polysaccharide.

Damit Gase wie CO₂ oder das Rauchgas nicht entweichen, kann der Bioreaktor hermetisch abgeschlossen sein.

In einer weiteren Ausführungsform kann der Bioreaktor eine Ausrichtung aufweisen, welche linear, kreisförmig, teilkreisförmig, elliptisch, teilelliptisch, winkelig oder schräg ist. Mit Hilfe dieser Ausrichtungen kann der Bioreaktor beispielsweise an die örtlichen Gegebenheiten angepasst sein. So können halbkreisförmige oder halbelliptische Anordnungen gewährleisten, dass über den Tagesverlauf hinweg die Algen optimal mit Sonnenlicht versorgt werden. Weiterhin kann die schräge Ausrichtung anhand des Sonnenstandes ausgerichtet sein.

Um die Ausrichtung den Bedingungen anzupassen, kann die Ausrichtung mittels einer Steuer- oder Regeleinheit und einer zugeordneten Stelleinheit änderbar sein. Somit kann die Algenproduktion optimiert werden. Die vorliegenden Bedingungen können Sonnenstand, Temperatur oder ein Beschattungsgrad sein.

In einer weiteren Ausführungsform ist die Ausrichtung des Bioreaktors anhand eines Sensorsignals geregelt, wobei der Sensor insbesondere eine Lichtintensität, einen Sonnenstand, eine Wellenlänge des Lichts und/oder Bedingungen in der Flüssigkeits-Algen-Mischung bestimmt. Somit kann automatisch anhand bestimmter Sollwerte die Ausrichtung des Bioreaktors eingestellt werden. Insbesondere ist eine Nachführung des Bioreaktors anhand des Sonnenstandes oder der Dichte der Algen in der Flüssigkeits-Algen-Mischung realisierbar.

In einer weiteren Ausprägung der Erfindung kann die Aufgabe gelöst werden durch ein Hausdach, welches einen zuvor beschriebenen Bioreaktor aufweist. Somit kann ein Bioreaktor die häufig ungenutzte Hausdachfläche zum Erzeugen von Biomasse verwenden.

In einem weiteren Aspekt der Erfindung wird die Aufgabe gelöst durch ein Bioreaktorsystem, welches zwei oder mehr Bioreaktoren aufweist. Somit kann eine Nutzfläche optimal mit Bioreaktoren belegt werden, sodass eine optimierte Ausbeute erfolgen kann.

Um auf Umweltgegebenheiten wie beispielsweise Sonnenstand einzugehen, kann ein Abstand zwischen zwei Bioreaktoren änderbar ausgestaltet sein, wobei die Änderung des Abstands mittels einer Steuer- oder Regeleinheit und einer zugeordneten Stelleinheit erfolgt.

In einem weiteren Aspekt ist die Änderung des Abstands der Bioreaktoren anhand eines Sensorsignals geregelt, wobei der Sensor insbesondere eine Lichtintensität, einen Sonnenstand, eine Wellenlänge des Lichts und/oder Bedingungen in der Flüssigkeits-Algen-Mischung bestimmt. Als Bedingungen in der Flüssigkeits-Algen-Mischung kann insbesondere eine Temperatur in Frage kommen.

In einem weiteren Aspekt der Erfindung wird die Aufgabe gelöst durch ein Gewächshaus, welches einen zuvor beschriebenen Bioreaktor oder ein zuvor beschriebenes Bioreaktorsystem aufweist. Somit können die natürlichen Vorteile eines Gewächshauses, wie insbesondere die Temperaturstabilität, mit den Vorteilen der Bioreaktoren kombiniert werden.

In einem weiteren Aspekt wird die Aufgabe gelöst mittels Algen, welche mittels des zuvor beschriebenen Bioreaktorsystems oder des Gewächshauses oder Hausdachs oder Bioreaktors hergestellt sind. Somit können Algen bereitgestellt werden, welche unter optimierten Kultivierbedingungen herangezogen wurden.

In einem weiteren Aspekt der Erfindung wird die Aufgabe gelöst durch Algenumwandlungsprodukte, insbesondere Verbrennungskraftstoffe, welche aus den zuvor beschriebenen Algen hergestellt sind.

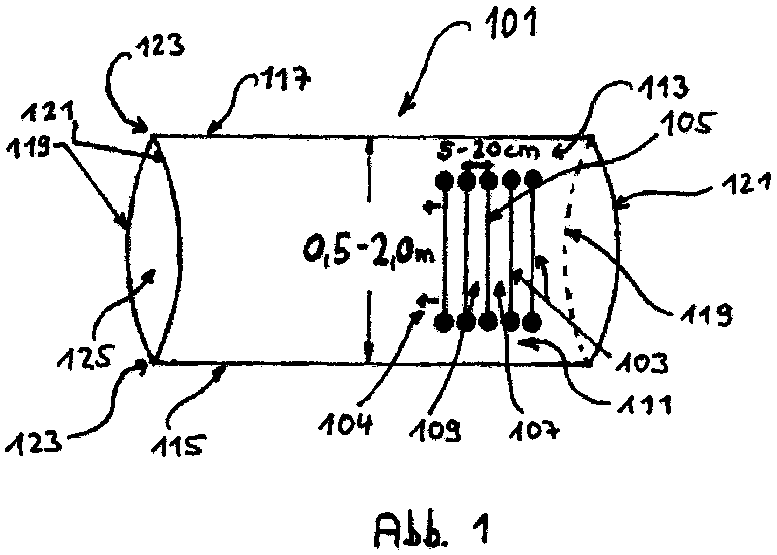

Im Weiteren wird die Erfindung anhand von Ausführungsbeispielen unter Bezugnahme auf die Zeichnung beschrieben. Es zeigen
- Figur 1: eine schematische Darstellung eines schlauchförmigen Foliensystems mit mehreren Trennverbindungen und dadurch separierten Bereiche,
- Figur 2: eine schematische Darstellung eines aus dem Foliensystem hergestellten Bioreaktors zum Kultivieren und Ernten von Algen,

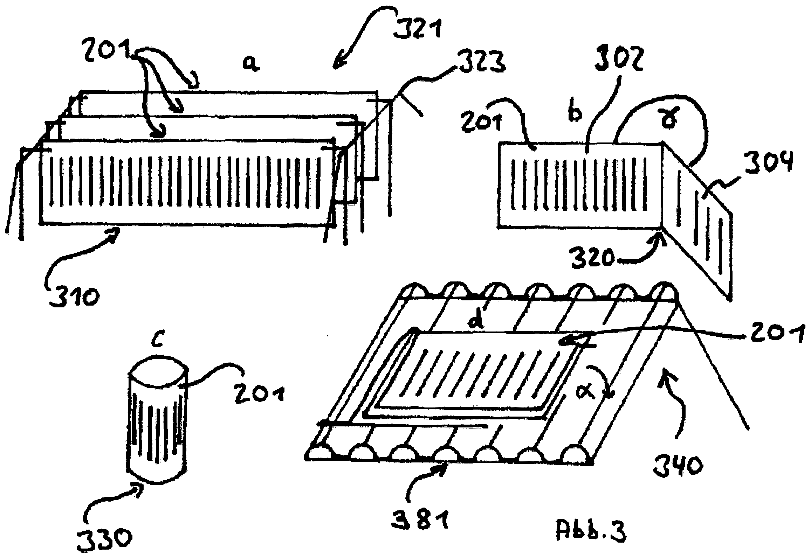

- Figur 3a: eine schematische Darstellung von linearen Bioreaktoren, welche auf einem Gestell hintereinander angeordnet sind,
- Figur 3b: eine schematische Darstellung eines linearen Bioreaktors mit abgewinkeltem Bioreaktorbereich,
- Figur 3c: eine schematische Darstellung eines elliptisch angeordneten Bioreaktors,
- Figur 3d: eine schematische Darstellung eines Hausdaches mit angeordnetem Bioreaktor und

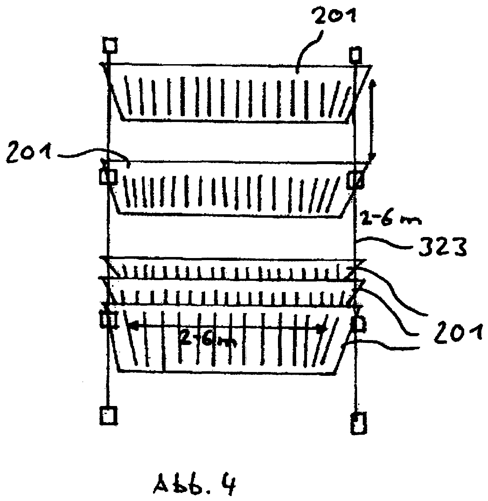

- Figur 4: eine schematische Darstellung mehrerer hintereinander auf einem Gestell angeordneter linearer Bioreaktoren

Das Foliensystem 101 ist schlauchförmig ausgestaltet. Das Foliensystem 101 weist mehrere vertikale Trennverbindungen 103, 105 (exemplarisch gekennzeichnet) auf. Die Trennverbindungen 103, 105 setzen sich in Trennverbindungspfeilrichtung 104 fort. Durch jede Trennverbindung 105 bilden sich separierte Bereiche 107, 109 (exemplarisch gekennzeichnet).

Durch die schlauchförmige Form des Foliensystems bilden die separierten Bereiche 107, 109 Kammern mit einem Schlauchmantel. Unterhalb der Trennverbindungen 103, 105 ist eine erste ungehinderte Verbindung 111, welche sich horizontal über das gesamte Foliensystem 101 erstreckt. Oberhalb der Trennverbindungen 103, 105 ist eine zweite ungehinderte Verbindung 113, welche sich ebenfalls horizontal über das gesamte Foliensystem 101 erstreckt.

An Breitenaußenkanten 115, 117 sind die zwei Folieninnenflächen zusammengeschweißt, sodass sich Breitenaußenverbindungen 123 bilden.

Eine sichtbare Folienschlauchinnenfläche 125 wird durch Längenaußenkanten 119 begrenzt.

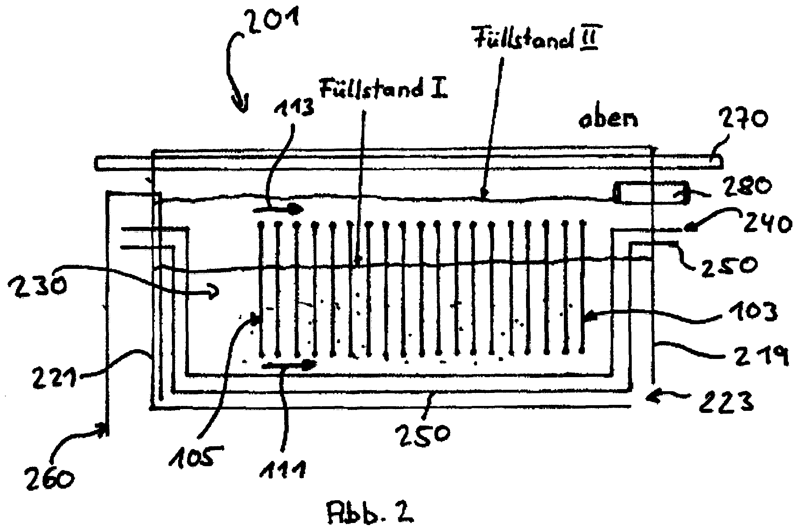

Durch Verschweißen der Längenaußenkanten 119 mit Längenaußenkanten 121 wird die Schlauchhülle des Bioreaktors 201 (vgl. Figur 2) hergestellt.

Die Schlauchhülle ist mit einem Befestigungsrohr 270 so versehen, dass das Schlauchsystem hängend angeordnet ist. Über die Bioreaktoröffnung sind ein Kühl-Wärm-Schlauch 240, ein poröser Gasversorgungsschlauch 250 und ein Ernteschlauch 260 eingeführt. Zusätzlich ist der Bioreaktor 201 mit einem Überlaufventil 280 versehen, wobei das Überlaufventil 280 als Einwegeventil ausgestaltet ist.

Nach dem hermetischen Verschließen wird der Bioreaktor 201 mit einer Algenwassermischung bis zu einem Füllstand I befüllt. Der Bioreaktor 201 wird an einem sonnigen Ort aufgestellt und über die Schläuche 240, 250, ggf. 260 mit allen notwendigen Nährstoffen und Umweltbedingungen versorgt.

Durch das Algenwachstum und gegebenenfalls durch nachgefülltes Wasser ist nach einer Kultivierungszeit ein Füllstand II erreicht. Zu diesem Zeitpunkt erfolgt die Ernte der Algen über den Ernteschlauch 260.

Der dargestellte Bioreaktor 201 wird in verschiedenen Konfigurationen entsprechend den Platzbedingungen aufgestellt.

In einer ersten Ausführungsform 321 sind über die Befestigungsrohre 270 mehrere Bioreaktoren 201 an einem Gestell 323 aufgehängt. Über ein Regler-, Sensoren- und Stellmotorensystem (hier nicht dargestellt) werden die Bioreaktoren 201 zueinander beabstandet und ggf. gedreht, sodass das Sonnenlicht die Bioreaktoren 201 optimal mit Sonnenlicht versorgt.

In einer weiteren Variante wird der Bioreaktor 201 mit einem Knick 320 aufgestellt. Dabei bilden ein erster Bioreaktorenabschnitt 302 und ein zweiter Bioreaktorenabschnitt 304 einen Winkel 360°-γ.

In einer dritten Variante ist der Bioreaktor 201 in einer Ellipse 330 angeordnet.

In einer vierten Variante ist der Bioreaktor 201 auf einem Hausdach 381 angebracht. Durch eine Schräge 340 des Hausdachs 381 sind für den Breitengrad die Sonneneinstrahlbedingungen für den Bioreaktor 201 optimiert.

### Bezugszeichenliste:

- 101: Foliensystem
- 103/105: Trennverbindung
- 104: Trennverbindungspfeilrichtung
- 107/109: separierte Bereiche
- 111: erste ungehinderte Verbindung
- 113: zweite ungehinderte Verbindung
- 115/117: Breitenaußenkanten
- 119/121: Längenaußenkanten
- 123: Außenverbindung
- 125: Folienschlauchinnenfläche
- 201: Bioreaktor
- 219/221: verbundenen Längenaußenkanten
- 223: Bioreaktoröffnung
- 230: Flüssigkeits-Algen-Mischung
- 240: Kühl-/Wärm-Schlauch
- 250: poröser Gasversorgungsschlauch
- 260: Ernteschlauch
- 270: Befestigungsrohr
- 280: Überlaufventil
- 302: erster Bioreaktorenabschnitt
- 304: zweiter Bioreaktorenabschnitt
- 310: linearer Bioreaktor
- 320: Knick
- 321: Bioreaktorsystem
- 323: Gestell
- 330: Ellipse
- 340: Schräge
- 381: Hausdach

## Patentansprüche

1. Foliensystem (101) zum Bereitstellen eines Bioreaktors (201) zum Kultivieren photosynthetisch aktiver Organismen, insbesondere zum Kultivieren in wässrigen Lösungen, aus einem transparenten Folienmaterial, insbesondere aus einem Polyethylen, wobei das Foliensystem zwei einander gegenüberliegende Innenseiten und die einander gegenüberliegenden Innenseiten eine Trennverbindung (103, 105) aufweisen, sodass zwei separierte Bereiche (107, 109) ausgebildet sind, und die Trennverbindung unvollständig ist, sodass die zwei separierten Bereiche eine erste ungehinderte Verbindung (111) aufweisen, wobei die gegenüberliegenden Innenseiten n unvollständige Trennverbindungen aufweisen, wobei n eine natürliche Zahl > 1 ist, sodass n+1 separierte Bereiche ausgebildet sind, welche insbesondere durch die erste Verbindung und/oder durch eine zweite ungehinderte Verbindung durchgehend verbunden sind, **dadurch gekennzeichnet, dass** das Foliensystem zum Herstellen von Bioreaktoren variabler Länge einsetzbar ist, wobei das Foliensystem eine endlose Länge aufweist, wobei das endlose Foliensystem von einer transportablen Foliensystemrolle abrollbar ist.

2. Foliensystem (101) nach Anspruch 1, **dadurch gekennzeichnet, dass** die Trennverbindung derart ausgestaltet ist, dass die zwei separierten Bereiche eine zweite ungehinderte Verbindung (113) aufweisen, welche durch die unvollständige Trennverbindung von der ersten unvollständigen Verbindung getrennt ist.

3. Foliensystem nach einem der vorhergehenden Ansprüche, **dadurch gekennzeichnet, dass** das Foliensystem eine Versorgungseinrichtung oder mehrere Versorgungseinrichtungen aufweist, insbesondere zum Versorgen der Algen mit CO₂, Rauchgas, Wärme, Kühlung oder Nährstoffen.

4. Foliensystem (101) nach einem der vorhergehenden Ansprüche, **dadurch gekennzeichnet, dass** die zwei Innenseiten je zwei Breitenaußenkanten (115, 117) und je zwei Längenaußenkanten (119, 121) aufweisen und die sich gegenüberliegenden Breitenaußenkanten der zwei gegenüberliegenden Innenseiten eine Außenverbindung (123) aufweisen, sodass insbesondere ein Folienschlauch mit einer Folienschlauchinnenfläche (125) gebildet ist und die Folienschlauchinnenfläche im Wesentlichen die zwei Innenseiten umfasst.

5. Foliensystem (101) nach einem der vorhergehenden Ansprüche, **gekennzeichnet durch** eine Breite zwischen 0,2 m und 6,0 m, insbesondere zwischen 0,5 m und 2,0 m.

6. Foliensystem (101) nach einem der vorhergehenden Ansprüche, **dadurch gekennzeichnet, dass** zwei benachbarte Trennverbindungen einen Abstand zwischen 2 cm und 200 cm aufweisen, wobei der Abstand insbesondere einen Wert zwischen 5 cm und 20 cm aufweist.

7. Foliensystem (101) nach einem der vorhergehenden Ansprüche **gekennzeichnet durch** eine Länge zwischen 0,5 m und 30.000 m, wobei die Länge insbesondere einen Wert zwischen 2 m und 10.000 m aufweist.

8. Foliensystem (101) nach einem der vorhergehenden Ansprüche, **dadurch gekennzeichnet, dass** das Folienmaterial einen Lichtleiter aufweist, durch welchen Licht in den Bioreaktor einleitbar ist, oder das Folienmaterial Wölbungen zum Vergrößern der Oberfläche aufweist.

9. Foliensystem (101) nach einem der vorhergehenden Ansprüche, **dadurch gekennzeichnet, dass** das Folienmaterial Nanopartikel und/oder Mikropartikel aufweist, welche auf physikalische und/oder chemische Eigenschaften des Folienmaterials einwirken.

10. Foliensystem (101) nach einem der vorhergehenden Ansprüche, **dadurch gekennzeichnet, dass** das Folienmaterial eine Beschichtung aufweist.

11. Foliensystem (101) nach einem der vorhergehenden Ansprüche, **dadurch gekennzeichnet, dass** das Folienmaterial und/oder die Beschichtung als Spektralfilter und/oder als teildurchlässiger Spiegel ausgestaltet ist.

12. Foliensystemrolle, **gekennzeichnet durch** ein Foliensystem nach einem der vorhergehenden Ansprüche, welches zu der Foliensystemrolle aufgerollt ist, sodass einem Benutzer beliebig lange Foliensysteme zum Herstellen eines Bioreaktors als Einzelnutzen zur Verfügung stehen.

13. Verfahren zum Herstellen eines Bioreaktors (201), **dadurch gekennzeichnet, dass** bei einem Foliensystem nach einem der Ansprüche 1 bis 11 die Längenaußenkanten mediendicht verbunden werden, sodass eine Flüssigkeits-Algen-Mischung (230) in dem Bioreaktor lagerbar ist.

14. Verfahren nach Anspruch 13, **dadurch gekennzeichnet, dass** vor dem mediendichten Verbinden ein Foliensystem von der Foliensystemrolle abgetrennt wird, wobei das Abtrennen von Foliensystemen insbesondere für unterschiedliche Längen der Foliensysteme erfolgt.

15. Verfahren nach Anspruch 13 oder 14, **dadurch gekennzeichnet, dass** vor dem mediendichten Verbinden ein oder mehrere Versorgungsschläuche in den Bioreaktor eingebracht werden.

## Claims

1. A film system (101) for providing a bioreactor (201) for cultivating photosynthetically active organisms, in particular for cultivation in aqueous solutions, made of a transparent film material, in particular a polyethylene, wherein the film system comprises two opposing inner sides and the opposing inner sides comprise a separating connection (103, 105), so that two separate regions (107, 109) are formed, and the separating connection is not continuous so that the two separated regions comprise a first unhindered connection (111), wherein the opposing inner sides comprise n non-continuous separating connections, wherein n is a natural number > 1, so that n+1 separated regions are formed, which are continuously connected in particular by way of the first connection and/or by way of a second unhindered connection, **characterized in that** the film system can be used for manufacturing bioreactors of variable lengths, wherein the film system has an endless length, wherein the endless film system can be unrolled from a transportable film system roll.

2. The film system according to claim 1, **characterized in that** the separating connection is designed in such a manner that the two separated regions have a second unhindered connection (113), which is separated from the first non-continuous connection by the non-continuous separating connection.

3. The film system according to one of the afore-mentioned claims, **characterized in that** the film system comprises a supply device or several supply devices, in particular for supplying the algae with CO2, flue gas, heat, cooling or nutrients.

4. The film system (101) according to one of the afore-mentioned claims, **characterized in that** the two inner sides comprise respectively two widthwise outer edges (115, 117) and respectively two lengthwise outer edges (119, 121) and the opposing widthwise outer edges of the two opposing inner sides comprise an outer connection (123), so that in particular a film tube is formed with a film tube inner surface (125) and the film tube inner surface substantially includes the two inner sides.

5. The film system (101) according to one of the afore-mentioned claims, **characterized by** a width between 0.2 m and 6.0 m, in particular between 0.5 m and 2.0 m.

6. The film system (101) according to one of the afore-mentioned claims, **characterized in that** two adjacent separating connections have a separation distance between 2 cm and 200 cm, the distance amounting in particular to between 5 cm and 20 cm.

7. The film system (101) according to one of the afore-mentioned claims, **characterized by** a length between 0.5 m and 30,000 m, wherein the length amounts in particular to between 2 m and 10,000 m.

8. The film system (101) according to one of the afore-mentioned claims, **characterized in that** the film material comprises a light conductor through which light can be introduced into the bioreactor, or the film material comprises bulges for increasing the surface.

9. The film system (101) according to one of the afore-mentioned claims, **characterized in that** the film material comprises nanoparticles and/or microparticles, which act on physical and/or chemical properties of the film material.

10. The film system (101) according to one of the afore-mentioned claims, **characterized in that** the film material comprises a coating.

11. The film system (101) according to one of the afore-mentioned claims, **characterized in that** the film material and/or the coating are designed as a spectral filter and/or a semi-transparent mirror.

12. A film system roll, **characterized by** a film system according to one of the afore-mentioned claims, which is rolled up to form the film system roll, so that film systems of any desired length are made available as individual blanks to a user for manufacturing a bioreactor.

13. A method for manufacturing a bioreactor (201), **characterized in that** in a film system according to one of the claims 1 to 11, the lengthwise outer edges are impermeably joined, so that a liquid algae mixture (230) can be stored in the bioreactor.

14. The method according to claim 13, **characterized in that** a film system is separated from the film system roll before impermeable joining, wherein the separation of film systems is carried out in particular for different lengths of the film systems.

15. The method according to claim 13 or 15, **characterized in that** one or several supply tubes are introduced into the bioreactor before impermeable joining.

## Revendications

1. Système de feuilles (101) pour mettre à disposition un bioréacteur (201) pour la culture d'organismes photo synthétiquement actifs, en particulier pour la culture en solutions aqueuses, composé d'un matériau de feuille transparent, en particulier d'un polyéthylène, où le système de feuilles comporte deux côtés intérieurs opposés et les côtés intérieurs opposés comportent une liaison de séparation (103, 105), de sorte que deux régions séparées (107, 109) sont formées, et la liaison de séparation est incomplète, de sorte que les deux régions séparées présentent une première liaison non entravée (111), où les côtés intérieurs opposés comprennent n liaisons de séparation incomplètes, où n est un nombre naturel > 1, de sorte que n+1 régions séparées sont formées, qui sont reliées en continu en particulier par la première liaison et/ou par une seconde liaison non entravée, **caractérisé en ce que** le système de feuilles peut être utilisé pour la fabrication de bioréacteurs de longueurs variables, où le système de film présente une longueur sans fin, où le système de feuilles sans fin peut être déroulé d'un rouleau de système de feuilles transportable.

2. Système de feuilles (101) selon la revendication 1, **caractérisé en ce que** la liaison de séparation est configurée de telle manière que les deux régions séparées comprennent une seconde liaison non entravée (113) séparée de la première liaison incomplète par la liaison de séparation incomplète.

3. Système de feuilles (101) selon l'une des revendications précédentes, **caractérisé en ce que** le système de feuilles comporte un dispositif d'alimentation ou plusieurs dispositifs d'alimentation, en particulier pour alimenter les algues en CO2, gaz de fumée, chaleur, refroidissement ou nutriments.

4. Système de feuilles (101) selon l'une des revendications précédentes, **caractérisé en ce que** les deux côtés intérieures comprennent respectivement deux bords extérieurs dans le sens de la largeur (115, 117) et respectivement deux bords extérieurs dans le sens de la longueur (119, 121) et les bords extérieurs opposés dans le sens de la largeur des deux côtés intérieurs opposés comprennent une liaison extérieure (123), de sorte qu'un film tubulaire avec une surface intérieure de film tubulaire (125) est formé et la surface intérieure de film tubulaire comporte essentiellement les deux côtés intérieurs.

5. Système de feuilles (101) selon l'une des revendications précédentes, **caractérisé par** une largeur comprise entre 0,2 m et 6,0 m, en particulier entre 0,5 m et 2,0 m.

6. Système de feuilles (101) selon l'une des revendications précédentes, **caractérisé en ce que** deux liaisons de séparation adjacentes présentent un écart compris entre 2 cm et 200 cm, où l'écart a en particulier une valeur comprise entre 5 cm et 20 cm.

7. Système de feuilles (101) selon l'une des revendications précédentes, **caractérisé par** une longueur comprise entre 0,5 m et 30 000 m, où la longueur a en particulier une valeur comprise entre 2 m et 10 000 m.

8. Système de feuilles (101) selon l'une des revendications précédentes, **caractérisé en ce que** le matériau de feuille comporte un conduit de lumière à travers lequel de la lumière peut être introduite dans le bioréacteur, ou bien le matériau comprend des voûtes pour agrandir la surface.

9. Système de feuilles (101) selon l'une des revendications précédentes, **caractérisé en ce que** le matériau de feuilles comprend des nanoparticules et/ou des microparticules qui agissent sur des propriétés physiques et/ou chimiques du matériau de feuilles.

10. Système de feuilles (101) selon l'une des revendications précédentes, **caractérisé en ce que** le matériau de feuilles comprend un revêtement.

11. Système de feuilles (101) selon l'une des revendications précédentes, **caractérisé en ce que** le matériau de feuilles et/ou le revêtement sont conçus comme des filtres spectraux et/ou des miroirs semi-transparents.

12. Rouleau de système de feuilles **caractérisé par** un système de feuilles selon l'une des revendications précédentes qui peut être enroulé sur un rouleau de système de feuilles, de sorte qu'un utilisateur a à sa disposition des systèmes de feuille de n'importe quelle longueur en tant qu'exemplaires individuels pour la fabrication d'un bioréacteur.

13. Procédé de fabrication d'un bioréacteur (201), **caractérisé en ce que** dans un système de feuilles selon l'une des revendications 1 à 11, les bords extérieurs dans le sens de la longueur sont reliés de manière imperméable, de sorte qu'un mélange de liquide et d'algues (230) peut être stocké dans le bioréacteur.

14. Procédé selon la revendication 13, **caractérisé en ce qu'**avant la réalisation de la liaison imperméable, un système de feuilles est séparé du rouleau de systèmes de feuilles, où la séparation de systèmes de feuilles est effectuée en particulier selon des longueurs de système de feuille différentes.

15. Procédé selon la revendication 13 à 14, **caractérisé en ce qu'**un ou plusieurs tuyaux d'alimentation sont introduits dans le bioréacteur avant la réalisation de la liaison imperméable.
